# EUROPÄISCHE PATENTSCHRIFT

(11) **EP 0 480 391 B1**
(45) Veröffentlichungstag und Bekanntmachung des Hinweises auf die Patenterteilung: **10.01.1996**
(21) Anmeldenummer: 91117171.8
(22) Anmeldetag: 09.10.1991
(51) Int. Cl.: C08F 6/14, A61K 9/22, A61K 47/32

(54) **Emulgatorfreie Emulsionspolymere in pharmazeutischen Zubereitungen mit verzögerter Wirkstofffreigabe und ihre Herstellung**
Emulsion-free emulsion polymers in sustained-release pharmaceutical compositions and their production
Polymères en émulsion sans émulsifiant dans les compositions pharmaceutiques à libération retardée et leur préparation

(30) Priorität: 10.10.1990 DE 4032096
(43) Veröffentlichungstag der Anmeldung: 15.04.1992
(73) Patentinhaber: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Göpferich, Achim Dr., Arlington , MA 02174 (US); Lee, Geoffrey William James Prof., W-6900 Heidelberg (DE); Ludwig, Horst Prof. Dr., W-6906 Leimen 1 (DE); Schäffler, Achim Dr., W-6121 Rothenberg (DE); Zierenberg, Bernd Dr., W-6530 Bingen 1 (DE)

(56) Entgegenhaltungen:
- EP-A- 0 086 997

## Beschreibung

Die vorliegende Erfindung betrifft von Emulgatoren befreite Emulsionspolymerisate, ihre Herstellung und ihre Verwendung in pharmazeutischen Zubereitungen mit verzögerter Wirkstofffreigabe.

Bestimmte Emulsionspolymerisate - wie z.B. - emulsionspolymerisierte Ester der Acrylsäure oder Methacrylsäure - sind in den vergangenen Jahren unverzichtbare Hilfsmittel bei der Herstellung von Pharmazeutika geworden, bei denen für einen Wirkstoff eine Darreichungssform mit verzögerter Wirkstofffreigabe angestrebt wird [H. Determann und R. Lotz, Pharmazeutische Industrie 32 (1970) 469].

Derartige Depotformen erlauben über einen längeren Zeitraum eine gleichbleibende Wirkstofffreisetzung und gestatten es somit, die Anzahl der täglich zu verabreichenden Arzneimittelgaben zu verringern und somit das Therapieschema zu vereinfachen.

Im Laufe der Entwicklung dieser Depotformen sind neben einer Vielzahl Tabletten und Kapseln u.a. auch wirkstoffhaltige Pflaster mit kontrollierter Wirkstofffreigabe beschrieben worden.

Für die Herstellung derartiger Depotformen sind aus dem Stand der Technik ebenfalls zahlreiche Verfahren bekannt.

Zum Beispiel kann die Herstellung in der Weise erfolgen, daß man das Emulsionspolymerisat vom Suspensionsmittel befreit, das so isolierte Polymer mit dem Wirkstoff in einem geeigneten - organischen - Lösungsmittel löst, anschließend das Lösungsmittel abdampft und das feste wirkstoffhaltige Polyacrylat bei einer unter der Glastemperatur des Polymeren liegenden Temperatur vermahlt und das wirkstoffhaltige Polyacrylatpulver entweder unter Verwendung von Tablettierhilfsstoffen zu Tabletten oder in anderer Weise - beispielsweise durch Einbringen in Kapseln - verarbeitet.

Auch die Herstellung von wirkstoffhaltigen Pflastern ist aus dem Stand der Technik bekannt; sie erfolgt in analoger Weise [s. Europäische Patentschrift 20 905].

Für die Herstellung derartiger Zubereitungen geeignete Emulsionspolymere sind - wie schon eingangs erwähnt - Ester der Acryl- oder Methacrylsäure, wie z.B. die kommerziell verfügbaren Produkte mit der übergreifenden Produktbezeichnung Eudragit der Fa. Röhm, Darmstadt.

Derartige Emulsionspolymere werden in der Weise hergestellt, daß das wasserunlösliche Monomere mit Hilfe von Emulgatoren bzw. Tensiden in Wasser emulgiert wird und unter Anwendung üblicher Initiatoren die Polymerisation eingeleitet bzw. durchgeführt wird. Die bei dieser Art der Polymerisation anfallenden Polymerdispersionen sind in vielen Fällen direkt einsetzbar und in Form von Dispersionen verfügbar. Wirkstofffreigabesysteme die auf der Basis von derartigen Emulsionspolymerisaten hergestellt wurden, weisen jedoch den Nachteil auf, daß die Freigaberate des Wirkstoffs von der thermischen Behandlung des Wirkstofffreigabesystems beim Herstellungsprozess sowie von der Lagerung abhängen kann.

Es ist daher die Aufgabe der vorliegenden Erfindung Wirkstofffreigabesysteme auf der Basis von Emulsionspolymerisaten - insbesondere auf der Basis von Polyacrylsäureestern bzw. Polymethacrylsäureestern zur Verfügung zu stellen, die die oben beschriebene Abhängigkeit nicht aufweisen und außerdem auch nach längerer Lagerzeit und unterschiedlichen Lagerungsbedingungen eine im wesentlichen identische Freigabecharakteristik besitzen.

Überraschenderweise wurde nun gefunden, daß die Abhängigkeit der Freigaberate von der thermischen Vorbehandlung während des Herstellungsverfahrens, die im Freigabeverhalten von Wirkstoffreigabesystemen beobachtet werden konnten, auf die Anwesenheit des Emulgators zurückzuführen ist.

Es ist eine weitere Aufgabe der vorliegenden Erfindung Wirkstofffreigabesysteme auf Basis emulgatorfreier Emulsionspolymerisate sowie ein Verfahren zu ihrer Herstellung zur Verfügung zu stellen.

Erfindungsgemäß werden die bei der Herstellung von Wirkstofffreigabesystemen eingesetzten Emulsionspolymere vom Emulgator befreit und darausmit an sich bekannten Verfahren - die entsprechenden emulgatorfreien Wirkstofffreigabesysteme hergestellt.

Für das Entfernen des Emulgators bzw. der extrahierbaren Hilfsstoffe kommen mehrere Verfahren in Betracht. Prinzipiell wird das Emulsionspolymerisat mit einem Extraktionsmittel behandelt, in dem sich das Polymer selbst nicht löst.
Für die erfindungsgemäßen Extraktionsverfahren wird vorzugsweise Wasser eingesetzt.
Es ist jedoch in Abhängigkeit von der Art des eingesetzten Polymeren - möglich jedes andere Lösungsmittel bzw. Lösungsmittelgemisch einzusetzen, in dem das Polymere selbst nicht löslich ist.
Zur Durchführung der Extraktion besteht zum einen die Möglichkeit, den Emulgator aus der handelsüblichen Emulsion durch Ausfällen des Polymers zu entfernen.

Dies kann mittels üblicher Verfahren, wie z.B. durch Ausfällen mit einem geeigneten Lösungsmittel oder einer Säure, durch Aussalzen, Ausfrieren oder Extraktion erreicht werden.

Eine weitere Möglichkeit besteht darin, den Emulgator bzw. den/die Hilfsstoffe - nach Abtrennen des Dispersionsmittels - aus dem Polymer zu entfernen. Dazu bieten sich beispielsweise folgende Verfahren an:

Gleichgewichtsextraktion, Soxleth-Verfahren, Säulenextraktion oder Dialyse.

Alternativ sind andere Trennverfahren - wie z.B. die Zentrifugation geeignet.

Bevorzugt wird die Extraktion des getrockneten Acrylats (Eudragit NE 30 D^{R}).

Dabei wird das getrocknete Acrylat in einer geeigneten Apparatur zerkleinert bzw. gemahlen und mit Wasser, das ständig erneuert wird, extrahiert. Bevorzugt wird der Emulgator durch Ausfrieren des entsprechenden Emulsionspolymeren und anschließendes Auftauen sowie Waschen mit einem geeigneten Lösungsmittelvorzugsweise Wasser - entfernt.

Die Herstellung der Wirkstofffreigabesysteme ist - wie schon erwähnt - aus dem Stand der Technik bekannt und u.a. in der Deutschen Offenlegungsschrift 33 14 003 sowie in der Europäischen Patentschrift 0 086 997, auf die hiermit inhaltlich Bezug genommen wird, beschrieben.

Als Trägermaterial sind neben den eingangs erwähnten Polymeren auch solche Polymere geeignet, die sich nach dem Emulsionspolymerisationsverfahren herstellen lassen, wie z. B. PVC, Polylactide, Polystyrol, Polyvinylacetat, Polybutadien, Polyacrylnitril, Polyvinylpyrrolidon, Polyvinylester, Polyvinylether und deren Copolymere. Bevorzugt sind Polymere auf der Basis von Estern der Acryl- und/oder der Methacrylsäure.

Besonderes bevorzugt sind emulsionspolymerisierte Copolymerisate von Methyl- und/oder Ethylestern der Acryl- und Methacrylsäure.

Als pharmazeutische Wirkstoffe eignen sich neben Clonidin unter anderem Ranitidin, Cimetidin, Atenolol, Enalapril, Captopril, Nifedipin, Naproxen, Diclofenac, Diclofenac-Natrium, Piroxicam, Cefaclor, Diltiazem, Ketotifen, Ketotifen-hydrogenfumarat, Salbutamol, Propranolol, Amoxicillin, Triamteren, Norethisteron, Mestranol, Cefotoxamin, Cefotoxamin-Natrium, Ceftriaxon, Ceftriaxon-Dinatrium, Cefalexin, Dipyridamol, Alprazolam, Cefoxitin, Ciclosporin, Metoprololtartrat, Aciclovir, Sulindac, Clavulansäure, Methyldopa, Nicardipin, Pentoxifyllin, Glyceroltrinitrat, Timolol, Idebenon, Terfenadin, Tamoxifendihydrogencitrat, Prazosin, Doxorubicin, Amilorid, Amilorid°HCl, Hydrochlorothiazid, Dihydroergocornin, Dihydroergocorninmethansulfonat, Erythromycin, Erythromycinstearat, Triazolam, Latamoxef, Cromoglicinsäure, Ceftazidim, Clenbuterol, Bromhexin Oxytetracyclin, Dexamethason-21-isonicotinat, Sulfadiazin, Cimaterol, Aditoprim, Mederantil, Climazolam, Carprofen, Coffein und Acetylsalicylsäure - oder Vitamine - wie Z.B. Vitamin A, A₁, A₂, B₁, B₂, B₄, B₆, B₁₂, C (Ascorbinsäure), Ascorbylpalmitat und weitere pharmakologisch verträgliche Derivate der Ascorbinsäure, D, D₁, D₂, D₃, D₄, E, H, K, K₁, K₂, P und Q - oder Wirkstoffe - wie z.B. Avoparcin, Flavopholipol, Monensin, Monensin-Natrium, Salinomycin, Carbadox, Nitrovin und Olaquindox, sowie Wirkstoffe, wie sie in der Roten Liste 1991 (Editio Cantor Verlag für Medizin und Naturwissenschaften GmbH und Co. KG, Aulendorf/Württemb.) aufgeführt sind auf die hiermit inhaltlich Bezug genommen wird.

Zur Herstellung wird das Emulsionspolymere üblicherweise mit dem Wirkstoff in einem geeignetenorganischen - Lösungsmittel gelöst, das Lösungsmittel abgedampft und das feste, wirkstoffhaltige Polyacrylat entsprechend der geplanten Verwendung weiterverarbeitet.

### Erläuterung und Diskussion der Zeichnungen:

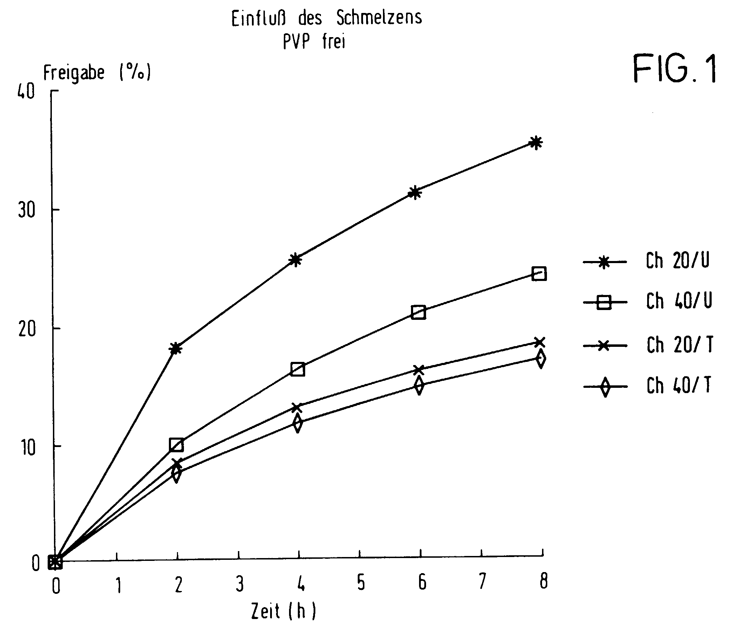

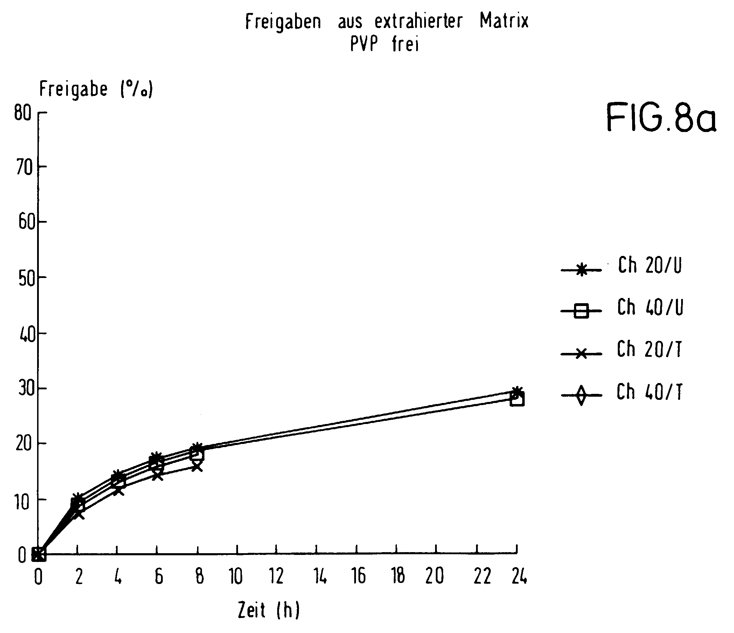

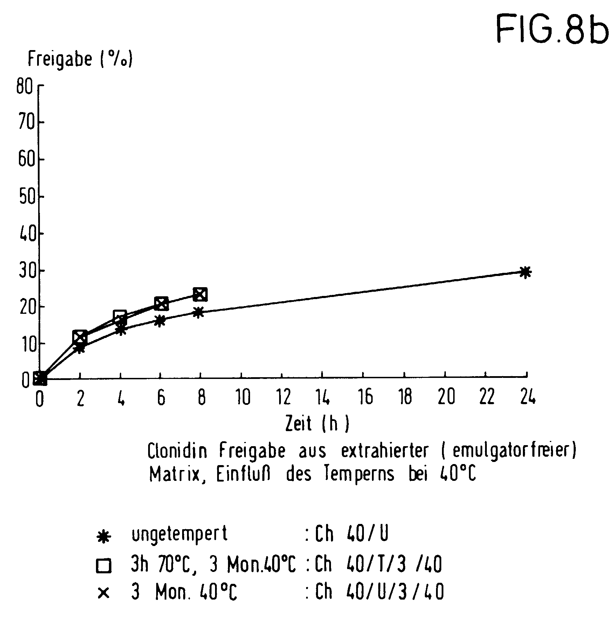

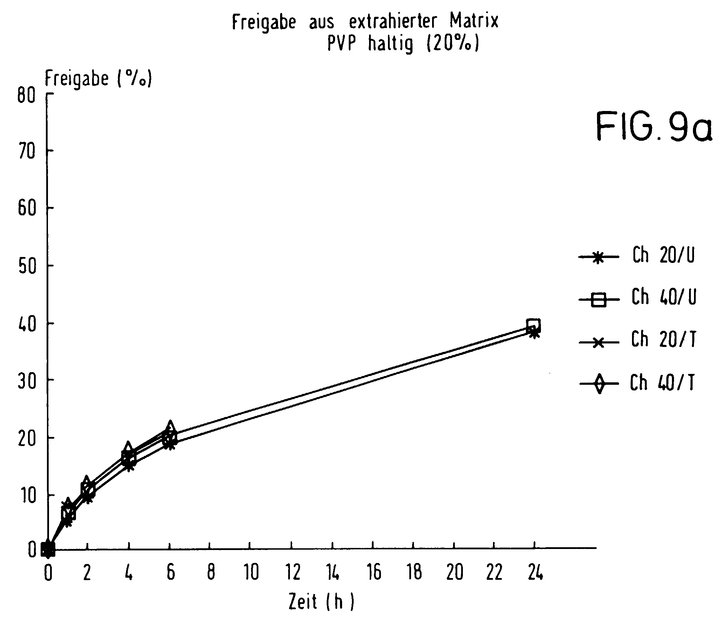

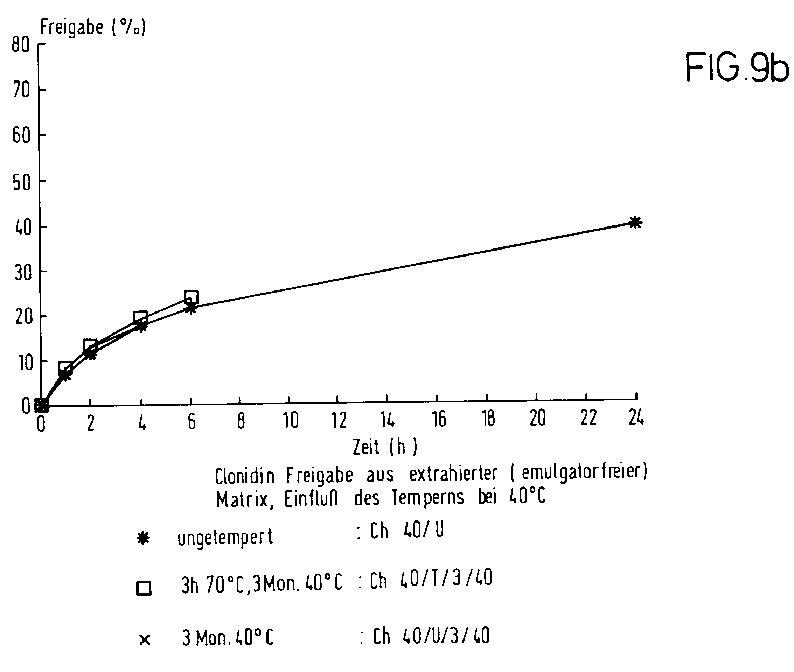

Die Figs. 1 bis 7 geben den Einfluß der thermischen Vorbehandlung und der Lagerzeit auf die Freigaberaten emulgatorhaltiger - auf Polyacrylatbasis (Eudragit) hergestellter - Wirkstofffreigabesysteme wieder. Der Wirkstoff ist in allen Fällen Clonidin. Demgegenüber zeigen die Figs. 8 und 9, daß die thermische Behandlung und Lagerzeit auf emulgatorfreie Wirkstofffreigabesysteme einen vernachlässigbar geringen Einfluß haben.

### Erläuterungen zu den Figs. 1 bis 13 im einzelnen:

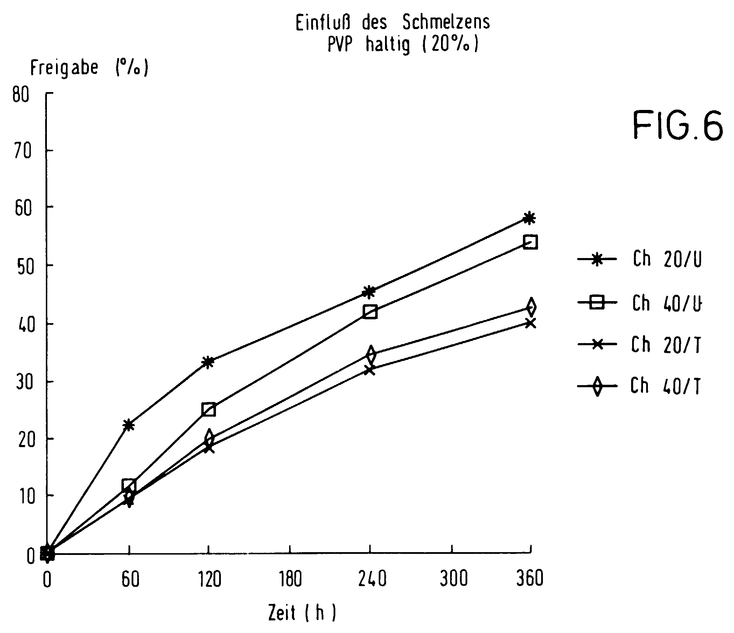

Die Figs. 1 bis 5 sowie 8 und 10 geben die Freigabecharakteristika verschiedener Wirkstoffzubereitungen bestehend aus 1,00 Gew.-% Clonidin und 99,00 Gew.-% Acrylat wieder. Die Figs. 6, 7 und 9 geben die Freigabecharakteristika entsprechender polyvinylpyrrolidonhaltiger Wirkstofffreigabesysteme wieder.

Als Acrylat wurde entsprechend den jeweiligen Angaben ein unbehandeltes bzw. ein vom Dispersionsmittel befreites Polyacrylat der Marke Eudragit NE 30 D^{R} der Firma Röhm, Darmstadt eingesetzt. Die Korngröße der jeweiligen Zubereitung liegt in einem Bereich von 315-400 µm. Die enthaltene Wirkstoffmenge beläuft sich auf 130 µg (= 100 %).

Die Meßwerte der jeweiligen Freigaberaten wurden wie folgt ermittelt:
der jeweils erste Meßwert wurde nach einer Behandlung der Probe über einen Zeitraum von 1 Stunde oder 2 Stunden mit künstlichem Magensaft - pH-Wert: 1,2 - ermittelt; alle weiteren Meßwerte wurden nach Behandlung der Proben - über den jeweils angegebenen Zeitraum - mit künstlichem Darmsaft - pH-Wert: 6,5 - mittelt.

### zu Fig. 1

Ch. 20/U steht für eine bei 20°C getrocknete nicht getemperte Probe.

Ch. 40/U steht für eine bei 40°C getrocknete nicht getemperte Probe.

Ch. 20/T steht für eine Probe, die bei 20°C getrocknet und über einen Zeitraum von 3 Stunden bei 70°C getempert worden war.

CH 40/T steht für eine Probe, die bei 40°C getrocknet und über einen Zeitraum von 3 Stunden bei 70°C getempert worden war.

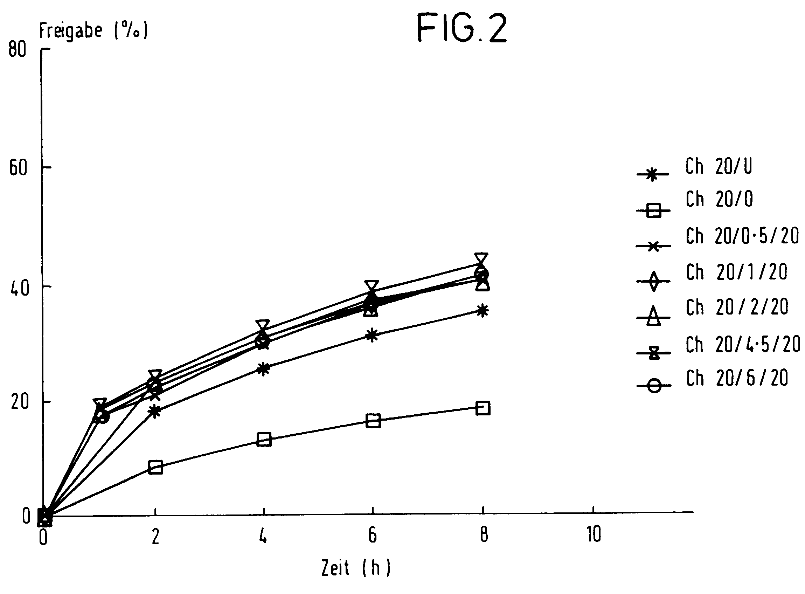

### zu Fig. 2:

Ch 20/U steht für eine ungetemperte Probe, die nach dem Trocknen bei 20°C vermessen worden war.

Ch 20/O steht für eine Probe, die bei 20°C getrocknet und über einen Zeitraum von 15 Stunden bei 70°C getempert und im Anschluß daran vermessen worden war.

Ch 20/0,5/20 steht für eine Probe, die bei 20°C getrocknet und über einen Zeitraum von 15 Stunden bei 70°C getempert und die - nach einer Lagerzeit von einem halben Monat bei 20°C - vermessen worden war.

Ch 20/1/20 steht für eine Probe, die bei 20°C getrocknet und über einen Zeitraum von 15 Stunden bei 70°C getempert und die - nach einer Lagerzeit von einem einen Monat bei 20°C - vermessen worden war.

Ch 20/2/20 steht für eine Probe, die bei 20°C getrocknet und über einen Zeitraum von 15 Stunden bei 70°C getempert und die - nach einer Lagerzeit von zwei Monaten bei 20°C - vermessen worden war.

Ch 20/4,5/20 steht für eine Probe, die bei 20°C getrocknet und über einen Zeitraum von 15 Stunden bei 70°C getempert und die - nach einer Lagerzeit von viereinhalb Monaten bei 20°C - vermessen worden war.

Ch 20/6/20 steht für eine Probe, die bei 20°C getrocknet und über einen Zeitraum von 15 Stunden bei 70°C getempert und die - nach einer Lagerzeit von sechs Monaten bei 20°C - vermessen worden war.

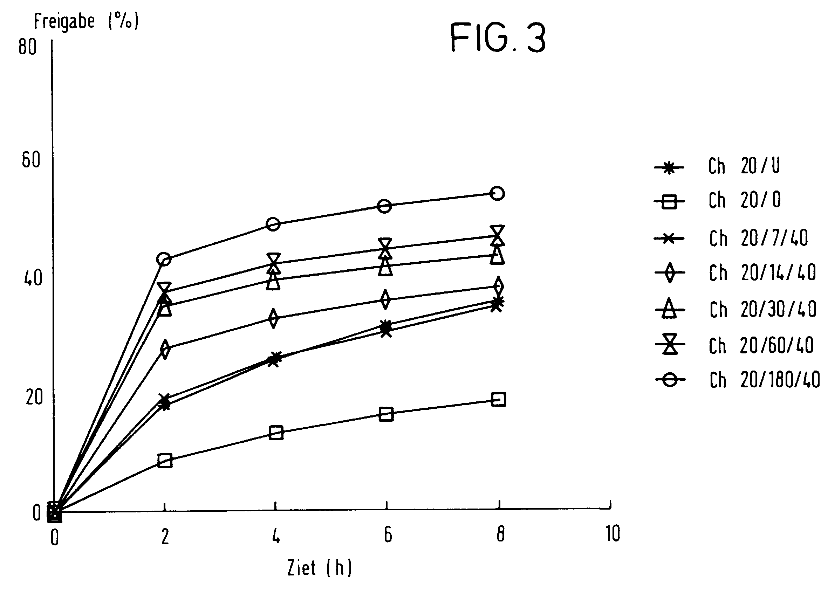

### zu Fig. 3:

Ch 20/U steht für eine ungetemperte Probe, die nach dem Trocknen bei 20°C vermessen worden war.

Ch 20/0 steht für eine Probe, die bei 20°C getrocknet und über einen Zeitraum von 15 Stunden bei 70°C getempert und im Anschluß daran vermessen worden war.

Ch 20/7/40 steht für eine Probe, die bei 20°C getrocknet und über einen Zeitraum von 15 Stunden bei 70°C getempert und die - nach einer Lagerzeit von 7 Tagen bei 40°C - vermessen worden war.

Ch 20/14/40 steht für eine Probe, die bei 20°C getrocknet und über einen Zeitraum von 15 Stunden bei 70°C getempert und die - nach einer Lagerzeit von 14 Tagen bei 40°C - vermessen worden war.

Ch 20/30/40 steht für eine Probe, die bei 20°C getrocknet und über einen Zeitraum von 15 Stunden bei 70°C getempert und die - nach einer Lagerzeit von 30 Tagen bei 40°C - vermessen worden war.

Ch 20/60/40 steht für eine Probe, die bei 20°C getrocknet und über einen Zeitraum von 15 Stunden bei 70°C getempert und die - nach einer Lagerzeit von 60 Tagen bei 40°C - vermessen worden war.

Ch 20/180/40 steht für eine Probe, die bei 20°C getrocknet und über einen Zeitraum von 15 Stunden bei 70°C getempert und die - nach einer Lagerzeit von 180 Tagen bei 40°C - vermessen worden war.

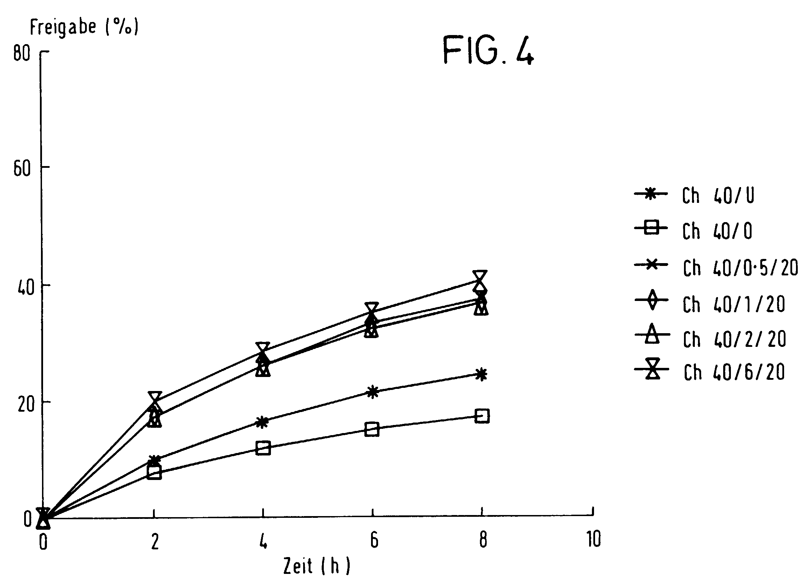

### zu Fig. 4:

Ch 40/U steht für eine ungetemperte Probe, die nach dem Trocknen bei 40°C vermessen worden war.

Ch 40/0 steht für eine Probe, die bei 40°C getrocknet und über einen Zeitraum von 15 Stunden bei 70°C getempert und im Anschluß daran vermessen worden war.

Ch 40/0,5/20 steht für eine Probe, die bei 40°C getrocknet und über einen Zeitraum von 15 Stunden bei 70°C getempert und die - nach einer Lagerzeit von einem halben Monat bei 20°C - vermessen worden war.

Ch 40/1/20 steht für eine Probe, die bei 40°C getrocknet und über einen Zeitraum von 15 Stunden bei 70°C getempert und die - nach einer Lagerzeit von einem Monat bei 20°C - vermessen worden war.

Ch 40/2/20 steht für eine Probe, die bei 40°C getrocknet und über einen Zeitraum von 15 Stunden bei 70°C getempert und die - nach einer Lagerzeit von zwei Monaten bei 20°C - vermessen worden war.

Ch 40/6/20 steht für eine Probe, die bei 40°C getrocknet und über einen Zeitraum von 15 Stunden bei 70°C getempert und die - nach einer Lagerzeit von sechs Monaten bei 20°C - vermessen worden war.

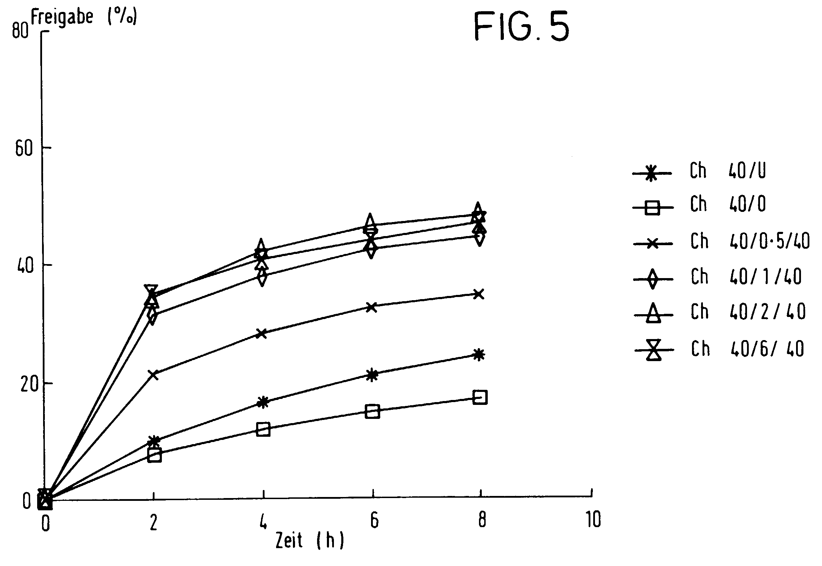

### zu Fig. 5:

Ch 40/U steht für eine ungetemperte Probe, die nach dem Trocknen bei 40°C vermessen worden war.

Ch 40/0 steht für eine Probe, die bei 40°C getrocknet und über einen Zeitraum von 15 Stunden bei 70°C getempert und im Anschluß daran vermessen worden war.

Ch 40/0,5/40 steht für eine Probe, die bei 40°C getrocknet und über einen Zeitraum von 15 Stunden bei 70°C getempert und die - nach einer Lagerzeit von einem halben Monat bei 40°C - vermessen worden war.

Ch 40/1/40 steht für eine Probe, die bei 40°C getrocknet und über einen Zeitraum von 15 Stunden bei 70°C getempert und die - nach einer Lagerzeit von einem Monat bei 40°C - vermessen worden war.

Ch 40/2/40 steht für eine Probe, die bei 40°C getrocknet und über einen Zeitraum von 15 Stunden bei 70°C getempert und die - nach einer Lagerzeit von zwei Monaten bei 40°C - vermessen worden war.

Ch 40/6/40 steht für eine Probe, die bei 40°C getrocknet und über einen Zeitraum von 15 Stunden bei 70°C getempert und die - nach einer Lagerzeit von sechs Monaten bei 40°C - vermessen worden war.

### zu Fig. 6:

Ch 20/U steht für eine polyvinylpyrrolidonhaltige, ungetemperte Probe, die nach dem Trocknen bei 20°C vermessen worden war.

Ch 40/U steht für eine polyvinylpyrrolidonhaltige, ungetemperte Probe, die nach dem Trocknen bei 40°C vermessen worden war.

Ch 20/T steht für eine polyvinylpyrrolidonhaltige Probe, die nach dem Trocknen bei 20°C über einen Zeitraum von drei Stunden bei 70°C getempert und anschließend vermessen worden war.

Ch 40/T steht für eine polyvinylpyrrolidonhaltige Probe, die nach dem Trocknen bei 40°C über einen Zeitraum von drei Stunden bei 70°C getempert und anschließend vermessen worden war.

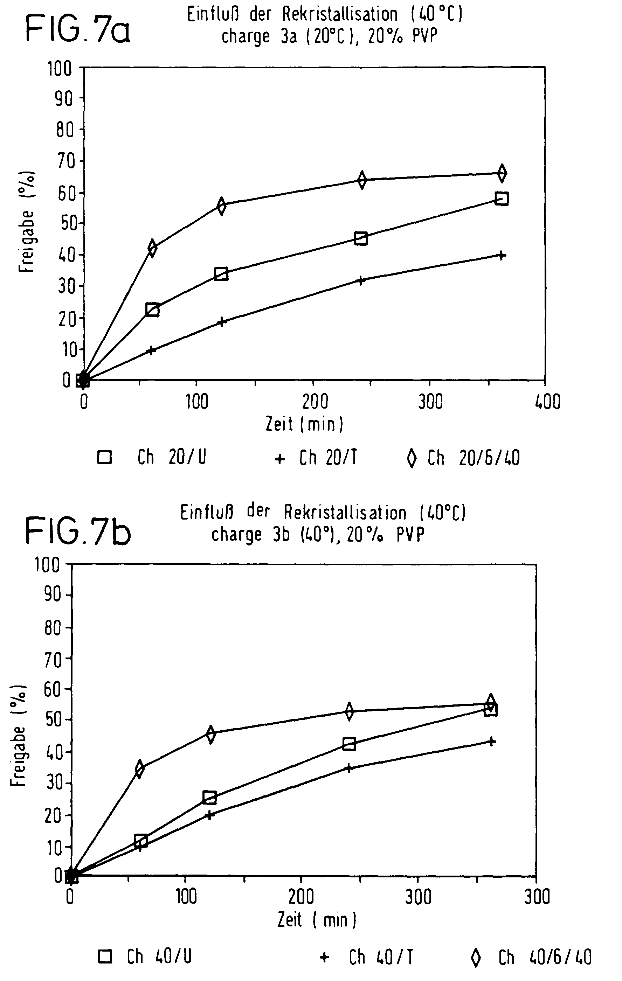

### zu Fig. 7a:

Ch 20/U steht für eine polyvinylpyrrolidonhaltige ungetemperte Probe, die nach dem Trocknen bei 20°C vermessen worden war.

Ch 20/T steht für eine polyvinylpyrrolidonhaltige Probe, die nach dem Trocknen bei 20°C 3 Stunden lang bei 70°C getempert und anschließend vermessen worden war.

Ch 20/6/40 steht für eine polyvinylpyrrolidonhaltige Probe, die nach dem Trocknen bei 20°C und dreistündigem Tempern bei 70°C sowie sechsmonatiger Lagerung bei 40°C vermessen worden war.

### zu Fig. 7b:

Ch 40/U steht für eine polyvinylpyrrolidonhaltige ungetemperte Probe, die - nach dem Trocknen bei 40°C - vermessen worden war.

Ch 40/T steht für eine polyvinylpyrrolidonhaltige Probe, die nach dem Trocknen bei 40°C 3 Stunden lang bei 70°C getempert und anschließend vermessen worden war.

Ch 40/6/40 steht für eine polyvinylpyrrolidonhaltige Probe, die nach dem Trocknen bei 40°C und dreistündigem Tempern bei 70°C sowie sechsmonatiger Lagerung bei 40°C vermessen worden war.

### zu Fig. 8a:

Ch 20/U steht für eine ungetemperte Probe, die nach dem Trocknen bei 20°C vermessen worden war.

Ch 40/U steht für eine ungetemperte Probe, die nach dem Trocknen bei 40°C vermessen worden war.

Ch 20/T steht für eine Probe, die nach dem Trocknen bei 20°C über einen Zeitraum von einer Stunde bei 70°C getempert und anschließend vermessen worden war.

Ch 40/T steht für eine Probe, die nach dem Trocknen bei 40°C über einen Zeitraum von einer Stunde bei 70°C getempert und anschließend vermessen worden war.

### Zu Figur 8b:

Ch 40/U steht für eine ungetemperte Probe, die nach dem Trocknen bei 40°C vermessen worden war.
Ch 40/T/3/40 steht für eine Probe, die 3 Stunden lang auf eine Temperatur von 70°C erwärmt worden war und nach einer dreimonatigen Lagerung vermessen wurde. Ch 40/U/3/40 steht für eine ungetemperte Probe, die nach einer dreimonatigen Lagerung bei 40°C vermessen worden war.

### Zu Figur 9a:

Ch 20/U steht für ungetemperte polyvinylpyrrolidonhaltige Probe, die - nach dem Trocknen bei 20°C - vermessen worden war.

Ch 40/U steht für eine ungetemperte polyvinylpyrrolidonhaltige Probe, die - nach dem Trocknen bei 40°C - vermessen worden war.

Ch 20/T steht für eine polyvinylpyrrolidonhaltige Probe, die nach dem Trocknen bei 20°C eine Stunde lang bei 70°C getempert und anschließend vermessen worden war.

Ch 40/T steht für eine polyvinylpyrrolidonhaltige Probe, die nach dem Trocknen bei 40°C eine Stunde lang bei 70°C getempert und anschließend vermessen worden war.

### Zu Figur 9b:

Ch 40/U steht für eine ungetemperte Probe. Ch 40/T/3/40 steht für eine Probe, die 3 Stunden lang auf eine Temperatur von 70°C erwärmt worden war und nach dreimonatiger Lagerung vermessen wurden.

Ch/46/U/3/40 steht für eine ungetemperte Probe, die nach einer dreimonatigen Lagerung bei 40°C vermessen worden war.

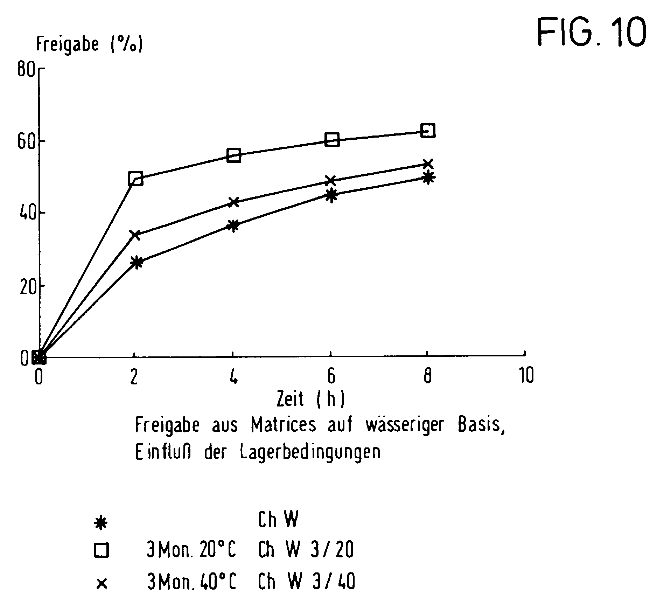

### Zu Figur 10:

Ch W steht für eine ungetemperte - direkt aus der wässrigen Dispersion hergestellte Probe - die bei einer Temperatur von 20°C getrocknet worden war und unmittelbar vermessen wurde.

Ch W/3/20 steht für eine ungetemperte - direkt aus der wässerigen Dispersion hergestellte Probe - die nach der Herstellung über einen Zeitraum von drei Monaten bei einer Temperatur von 20°C gelagert worden war und danach vermessen wurde.

Ch W/3/40 steht für eine ungetemperte - direkt aus der wässerigen Dispersion hergestellte Probe - die nach der Herstellung über einen Zeitraum von 3 Monaten bei einer Temperatur von 40°C gelagert worden war und anschließend vermessen wurde.

Fig. 1 zeigt die Freigaberaten von emulgatorhaltigen Zubereitungen, die nicht getempert bzw. 3 Stunden lang bei 70°C (Schmelzen des Emulgators) getempert worden waren und bei Temperaturen von 20°C bzw. 40°C getrocknet worden waren, als Funktion der Zeit. Aus Fig. 1 geht deutlich hervor, daß die Freigaberaten emulgatorhaltiger ungetemperter Matrices bei jeweils vorgegebener Zeitspanne die höchsten Werte annehmen, wobei die Freigaberaten der bei 20°C getrockneten Zubereitung vergleichsweise höher liegen als diejenigen der bei 40°C getrockneten Wirkstoffzubereitung.

Dagegen weisen die Freigaberaten der getemperten Proben deutlich kleinere Werte auf.

Fig. 2 zeigt die Freigaberaten von emulgatorhaltigen Wirkstoffzubereitungen, die jeweils bei 20°C getrocknet und 15 Stunden lang bei 70°C getempert worden waren und verschieden lange Zeiträume bei einer Temperatur von 20°C gelagert worden waren.

Aus Fig. 2 ist abzulesen, daß die jeweiligen Freigaberaten der lediglich getemperten Probe die niedrigsten Werte aufweisen, während die Freigaberaten der über einen längeren Zeitaum bei 20°C gelagerten Proben deutlich über denjenigen Werten der völlig unbehandelten Matrix und der nur getemperten Matrix liegen.

Fig. 3 zeigt die Freigaberaten von emulgatorhaltigen Wirkstoffzubereitungen, die jeweils bei 20°C getrocknet und 15 Stunden lang bei 70°C getempert worden waren und - im Unterschied zu Fig. 2 - verschieden lange Zeiträume bei einer Temperatur von 40°C gelagert worden waren.

Prinzipiell ist aus Fig. 3 ein analoges Verhalten der verschiedenen Wirkstoffzubereitungen nachweisbar.

Fig. 4 zeigt die Freigaberaten einer Wirkstoffzubereitung, die analog der Fig. 2 zugrundeliegenden Zubereitung behandelt - im Unterschied zu dieser jedoch bei 40°C getrocknet - worden war. Auch aus Fig. 4 ist zu entnehmen, daß die Freigaberaten mit wachsender Lagerungszeit der Proben ansteigen, so daß diejenige Probe, die 6 Monate lang bei 20°C gelagert worden war, die höchsten Freigaberaten aufweist. Demgegenüber besitzt die sofort vermessene Probe (0 Mon.) die kleinsten Freigabewerte.

Fig. 5 zeigt die Freigaberate einer Wirkstoffzubereitung, die analog der Fig. 3 zugrundeliegenden Zubereitung behandelt worden war - im Unterschied zu dieser jedoch bei 40°C getrocknet worden war. Prinzipiell sind in Fig. 5 die entsprechenden Tendenzen zu erkennen wie sie der Fig. 4 schon entnommen werden konnten.

Fig. 6 zeigt die Freigabecharakteristik einer emulgatorhaltigen polyvinylpyrrolidonhaltigen Matrix der Zusammensetzung: 1 Gew.-% Clonidin, 20 Gew.-% Polyvinylpyrrolidon und 79 % Acrylat (Eudragit NE 30 D^{R}) die bei 20 bzw. 40°C getrocknet worden war, in getemperter bzw. ungetemperter Form.

Die Messdaten der jeweiliegen Freigaberaten wurden nach folgender Behandlung der Proben aufgezeichnet:
1) 1 Stunde im künstlichen Magensaft
   - pH-Wert: 1,2;
2) 1 Stunde in künstlichem Darmsaft
   - pH-Wert: 6,5;
3) weitere 2 Stunden in künstlichem Darmsaft
   - pH-Wert: 6,5;
4) weitere 2 Stunden im künstlichem Darmsaft
   - pH-Wert: 6,5.

Auch aus Fig. 6 geht hervor, daß unbeschadet der Anwesenheit von Polyvinylpyrrolidon die Freigaberaten der getemperten Proben durchweg deutlich niedriger liegen, als diejenigen der ungetemperten Wirkstofffreigabesysteme.

Aus den Figuren 7a und 7b läßt sich der Einfluß verschiedener Trocknungsbedingungen ablesen. Die Zusammensetzung aller Proben besteht aus 1,00 Gew.-% Clonidin, 20,00 Gew.-% Polyvinylpyrrolidon und 79 % Acrylat. Die Behandlung aller Proben zur Aufnahme der Freigabecharakteristik erfolgte in Analogie zu dem in der Beschreibung zu Fig. 6 angegebenen Verfahren.

Fig. 7a zeigt die Freigabecharakteristik einer unbehandelten Probe, einer Probe, die drei Stunden bei 70°C aufgeschmolzen wurde, sowie einer Probe, die 3 Stunden bei 70°C aufgeschmolzen und 6 Monate bei einer Temperatur von 40°C gelagert worden war. Dabei wurden alle Proben bei einer Temperatur von 20°C getrocknet.

Fig. 7b zeigt die Freigabecharakteristika von Proben, die in Analogie zu dem in Fig. 7a behandelt wurden, mit dem Unterschied, daß die Trocknung bei einer Temperatur von 40°C durchgeführt wurde.

Fig. 8a gibt die Freigaberaten eines Wirkstofffreigabesystems wieder, dessen Matrix vom Emulgator befreit wurde.

Das Freigabesystem setzt sich aus 1,00 Gew.-% Clonidin und 99,00 Gew.-% emulgatorfreiem Acrylat zusammen. Die Messdaten der jeweiligen Freigaberaten wurden jeweils nach folgender Behandlung der Proben aufgezeichnet:
1) 2 Stunden in künstlichen Magensaft
   - pH-Wert: 1,2;
2) weitere 2 Stunden in künstlichem Darmsaft
   - pH-Wert: 6,5;
3) weitere 2 Stunden in künstlichem Darmsaft
   - pH-Wert: 6,5;
4) weitere 2 Stunden im künstlichen Darmsaft
   - pH-Wert: 6,5;
5) weitere 16 Stunden in künstlichem Darmsaft
   - pH-Wert: 6,5.

Wiedergegeben sind die Freigaberaten von Proben die bei unterschiedlichen Temperaturen (20°C und 40°C) getrocknet worden waren und die ungetempert bzw. nach einstündiger Temperung bei 70°C eingesetzt worden waren.

Figur 8b verdeutlicht das Freigabeverhalten eines Wirkstofffreigabesystems (Zusammensetzung wie unter Fig. 8a beschrieben) basierend auf einer emulgatorfreien Matrix nach 3-monatiger Lagerung einer Temperatur von 40°C.

Fig 9a zeigt die Freigabecharakteristika emulgatorfreier, polyvinylpyrrolidonhaltiger Wirkstofffreigabesysteme, die sich aus 1,00 Gew.-% Clonidin, 20,00 Gew.-% Polyvinylpyrrolidon und 79,00 Gew.-% Acrylat zusammensetzt. Die Messdaten der jeweiligen Freigaberaten wurden jeweils nach folgender Behandlung der Proben aufgezeichnet:
1) 1 Stunde im künstlichen Magensaft
   - pH-Wert: 1,2;
2) eine weitere Stunde im künstlichen Darmsaft
   - pH-Wert: 6,5;
3) weitere 2 Stunden im künstlichen Darmsaft
   - pH-Wert: 6,5;
4) weitere 2 Stunden im künstlichen Darmsaft
   - pH-Wert: 6,5;
5) weitere 18 Stunden in künstlichem Darmsaft
   - pH-Wert: 6,5.

Aufgezeichnet wurden die Freigaberaten von Proben, die bei unterschiedlichen Temperaturen getrocknet worden waren und die ungetempert bzw. nach einstündiger Temperung bei 70°C eingesetzt worden waren.

Fig 9b verdeutlicht das Freigabesystem eines Wirkstofffreigabesystems (Zusammensetzung wie unter 9a beschreiben) basierend auf einer emulgatorfreien Matrix nach dreimonatiger Lagerung bei einer Temperatur von 40°C.

Fig 10: zeigt das Freigabeverhalten eines direkt aus der wässerigen Dispersion hergestellten Wirkstofffreigabesystems (Zusammensetzung: 1 Gew.-% Clonidin, 99 Gew.-% Acrylat), das sofort nach der Herstellung vermessen sowie nach dreimonatiger Lagerung bei 20°C bzw. bei 40°C vermessen worden war.

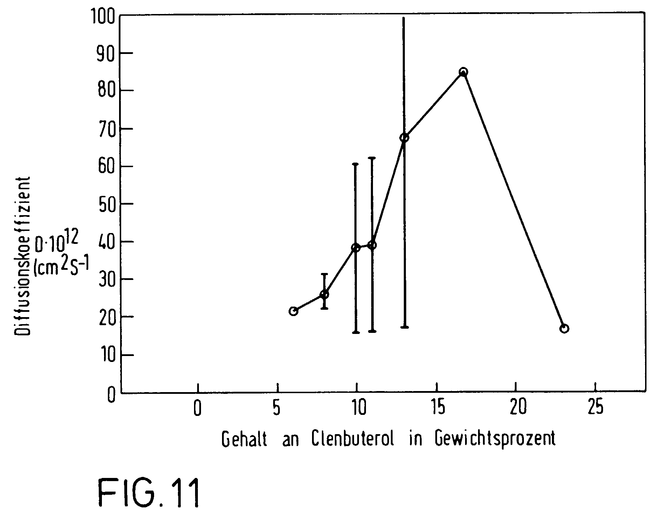

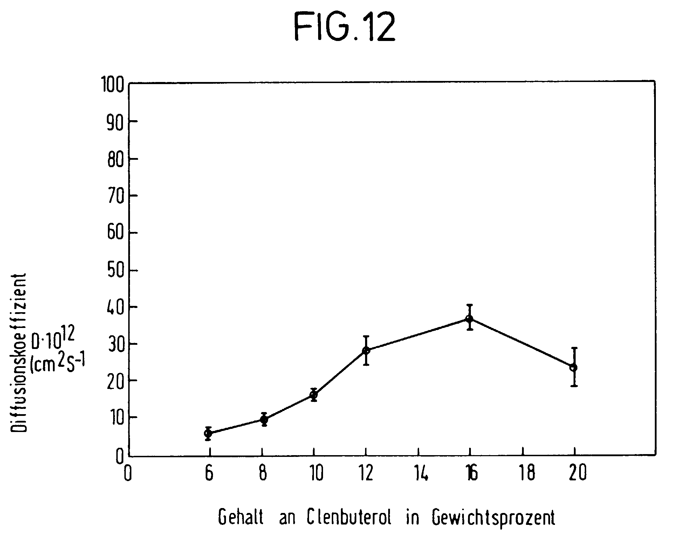

Fig. 11 und Fig. 12 geben die Abhängigkeit des Diffusionskoeffizienten von der Beladung einmal unter Verwendung einer nicht extrahierten Matrix (Fig. 11) und zu anderen unter Verwendung einer extrahierten Matrix (Fig. 12) wieder.

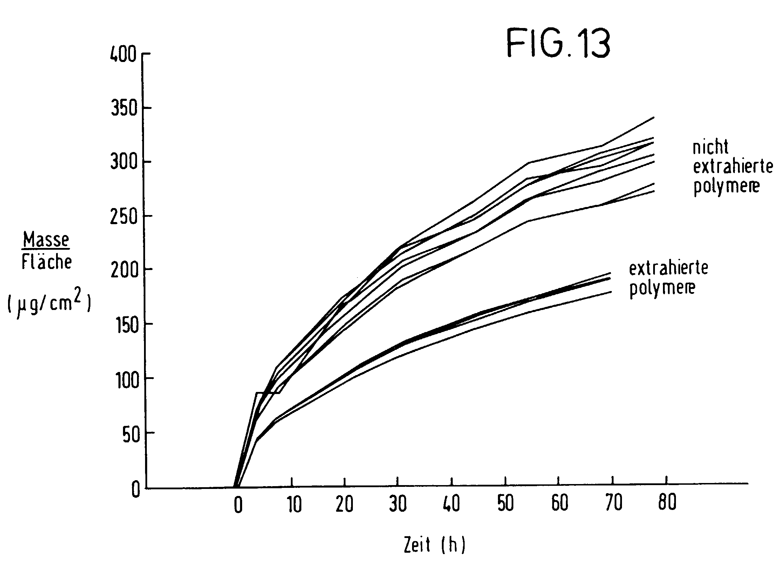

Fig. 13 gibt die Freigaberaten eines mit Clenbuterol (8 Gew.-%) beladenen Wirkstofffreigabesystems wieder. (Matrix: Eudragit NE 30 D).

Wie aus den Schaubildern der emulgatorhaltigen Wirkstofffreigabesystemen hervorgeht, sind die Freigaberaten von der thermischen Behandlung des Wirkstofffreigabesystems abhängig.

So liegen die Freigaberaten der ungetemperten Proben deutlich höher als diejenigen, die drei Stunden bei 70°C getempert worden waren.

Gleichzeitig ist zu erkennen, daß die Freigaberaten der - getemperten wie auch der ungetemperten - Proben, die bei 20°C getrocknet worden waren, größere Werte aufweisen, als die entsprechenden Werte derjenigen Proben, die bei 40°C getrocknet worden waren.

Die in den Figs. 2 bis 5 graphisch wiedergegebenen Freigabefunktionen belegen, daß unabhängig von der Trocknungstemperatur (20°C bzw. 40°C) und Lagerungstemperatur (20°C bzw. 40°C) bei getemperten Proben (70°C) folgender Trend festzustellen ist:

Die Freigaberaten derjenigen Proben, die sofort im Anschluß an das Tempern vermessen wurden weisen mit Abstand die niedrigsten Freigaberaten auf. Diese liegen deutlich unterhalb derjenigen Werte, die sich für die entsprechenden ungetemperten Proben ergeben. Mit wachsender Lagerungszeit steigen die Freigaberaten im allgemeinen unterschiedlich stark (in Abhängigkeit von der Trocknungs- und Lagerungstemperatur) an.

Die Figs. 6 und 7 zeigen den Einfluß des Emulgators auf die Freigaberaten in einem Polyacrylat/Polyvinylpyrrolidon-Gemisch, wie es kommerziell vertrieben wird. Die aus den Figs. 1 bis 5 gewonnenen Erkenntnisse werden auch durch die Freigaberaten dieser Systeme bestätigt.

So liegen die Freigaberaten der getemperten Proben unterhalb derjenigen der ungetemperten wobei wiederum eine Abhängigkeit von der Trocknungstemperatur nachweisbar ist. Auch in diesen Fällen steigen die Freigaberaten nach mehrmonatiger Lagerungszeit an.

Ein völlig anderes Bild ergibt sich dagegen bei den entsprechenden Untersuchungen der emulgatorfreien Wirkstofffreigabesysteme (Figs. 8a und 9a).

Unabhängig von den Trocknungsbedingungen weisen die Freigaberaten - sowohl in dem PVP-haltigen als auch in dem PVP-freien System - nur wenig unterschiedliche Werte auf, wobei auch die Differenz zu den Werten der getemperten Proben in beiden Fällen vernachlässigbar gering ausfällt.

Fig. 11 zeigt die Abhängigkeit des Diffusionskoeffizienten von der Beladung für eine nicht-extrahierte Matrix (Eudragit NE 30 D) - Fig. 12 zeigt diese Abhängigkeit für das entsprechende extrahierte Matrixmaterial. Dabei ist deutlich zu erkennen, daß für das gereinigte Polymer (Fig. 12) wesentlich kleinere Streuungen bezüglich des Diffusionskoeffzienten resultieren als für das entsprechende nichtextrahierte Acrylat.

Fig. 13 liefert ebenfalls einen deutlichen Beleg dafür, daß die Freigabe aus extrahierten Matrices wesentlich - bei einer geringeren Streuung zwischen den Kurven - langsamer erfolgt, als aus der entsprechenden nichtextrahierten Matrix.

Durch die Extraktion des Emulgators kann somit ein von der thermischen Behandlung des Freigabesystems unabhängiges Freigabeverhalten erreicht werden.

Weiterhin hat sich gezeigt (Figs. 8b und 9b), daß die erfindungsgemäß hergestellten Wirkstofffreigabesysteme eine verbesserte Haltbarkeit aufweisen, d.h., daß die Freigaberaten auch nach längerer Lagerung des Wirkstofffreigabesystems im wesentlichen unverändert geblieben sind.

Die der Anmeldung zugrundeliegenden Aufgaben werden durch das eingangs beschriebene Verfahren gelöst. - Wie bereits ausgeführt, werden bevorzugt Polymere auf der Basis von Estern der Acryl- und/oder der Methacrylsäure eingesetzt - besonders bevorzugt Eudragit NE 30 D^{R}. Das Molekulargewicht der geeigneten Polyacrylate sollte zweckmäßigerweise größer als 50.000 sein; vorzugsweise sollte das Molekulargewicht in einem Bereich von 100.000 bis 1.200 000 liegen, wobei ein Molekulargewicht von ca. 800.000 besonders bevorzugt wird. Hierzu sei angemerkt, daß derartige Molekulargewichte naturgemäß den Mittelwert einer entsprechenden Molekulargewichtsverteilung verkörpern. Als Emulgatoren kommen anionische, nicht-ionische, kationische und/oder amphotere Emulatoren in Frage, die in einer Vielzahl aus dem Stand der Technik bekannt sind [Kirk-Othmer, Enzyclopedia of Chemical Technology, 3. Auflage, Band 22, Verlag: John Wiley & Sons, New York N.Y., 1983, Seite 332 ff.].

Als Beispiele anionischer Emulgatoren seien genannt: Natrium-, Kalium- und Ammoniumsalze langkettiger aliphatischer Carbon- und Sulfonsäuren, Alkali-C₁₂₋₁₆ -alkylsulfate, Ethoxylate, Sulfate, Sulfonate oder Phosphate langkettiger aliphatischer Alkohole oder Alkylphenole sowie Ester von Sulfodicarbonsäuren.

Nicht-ionische Emulgatoren werden durch ethoxylierte Fettalkohole und Alkylphenole verkörpert wobei der Gehalt von Ethylenoxid-Einheiten in einem Bereich von 2 bis 150 mol/mol liegt.

Beispiele für kationische Emulgatoren sind u.a. Ammonium-, Phosphonium- und Sufonium-Verbindungen, die als hydrophoben Molekülteil mindestens eine lange aliphatische Kohlenwasserstoffkette enthalten.

Bevorzugt eignet sich das erfindungsgemäße Verfahren für Emulgatoren aus der Gruppe der - gegebenenfalls - derivatisierten Alkylphenole - besonders bevorzugt ethoxylierte Derivate des Nonylphenols.

Die Überprüfung, inwieweit die Lösungsmittel - bzw. das Waschwasser - mit denen bzw. dem das jeweils behandelte Emulsionspolymerisat gewaschen wurde extrakthaltig ist, kann analytisch unter Zuhilfenahme der spektroskopischen Daten erfolgen. Im Falle von Eudragit N 30D^{R} weist das UV-Spektrum eine starke Absorptionsbande bei 247 nm auf. Im Infrarotspektrum sind Absorptionsbanden bei 2900, 1100 und 830 cm⁻¹. Das 250 MHz-¹H-NMR-Spektrum [D₂O] zeigt - u.a. - charakteristische Peaks im aromatischen Bereich bei 6.8 und 7.2 ppm sowie ferner bei 3,7 ppm.

Die nachfolgende Methode erlaubt einen Nachweis des oben charakterisierten Emulgators bis zu einer Nachweisgrenze von 1 µg/ml: Zu jeweils 10.00 ml des zu prüfenden Waschwassers werden 2.00 ml einer 6°10⁻³N Lösung von KJ₃ in Wasser (hergestellt aus 0,810 g Jod bisublim. und 1,44 g KJ in 1 l Wasser) gegeben und UV-spektrometrisch die Extinktion bei 500 nm bestimmt (Referenz: Wasser).

Beispiele für geeignete Lösungsmittel u.a. für die extraktive Reinigung sind neben Wasser- in Abhängigkeit von der Löslichkeit des Emulgators und des Polymers - Alkohole - wie z. B. Methanol, Ethanol, Propanol oder Isopropylalkohol - oder mehrfunktionelle Alkohole - wie zum Beispiel Glykol und Glycerin - oder Ketone - wie z.B. Aceton oder Ethylmethylketon oder Mischungen von Ketonen mit Wasser - oder Ester - wie z.B Essigsäuremethyl- oder Essigsäureethylester - oder Ether - wie z.B. tert.-Butylmethylether, Glyme, Diglyme - oder cyclische Ether - wie z.B. Tetrahydrofuran oder Dioxan - oder Säureamide - wie z.B. Dimethylformamid oder Acetamid - oder Kohlenwasserstoffe - wie z.B. Pentan, Hexan, Heptan oder Erdölfraktionen, Benzol, Toluol oder Xylole - oder Halogenkohlenwasserstoffe - wie z.B. Dichlormethan, Chloroform, Tetrachlorkohlenstoff oder fluorierte Kohlenwasserstoffe - beispielsweise 1.1,1-Trifluorethan oder Mischungen der genannten Lösungsmittel, bzw. - sofern möglich - Mischungen mit Wasser.

Hinsichtlich der Extraktion der Polyacrylate werden Wasser und Alkohole als Extraktionsmittel bevorzugt wobei Wasser und Methanol besonders bevorzugt werden.

Mit dem erfindungsgemäß vorgeschlagenen Verfahren gelingt es, den Emulgator in einem Maße aus dem Emulsionspolymerisat zu entfernen, daß ein Nachweis beispielsweise mittels Differentialthermoanalyse nicht mehr möglich ist.

Der Gehalt an Restemulgator ist somit kleiner als 0,5 Gew.-% - bevorzugt kleiner 0,3 Gew.-% und besonders bevorzugt kleiner 0,1 Gew.-%.

Die eingangs genannten Aufgaben werden durch das im Beispiel 1 beschriebene Verfahren gelöst. Verschiedenartige, andere Ausgestaltungen des Verfahrens werden für den Fachmann aus der vorliegenden Beschreibung ersichtlich.

### Beispiel 1

30,0 g der wässerigen Eudragit NE 30 Dispersion wurden bei -18°C eingefroren und anschließend durch einfaches Stehenlassen bei Raumtemperatur aufgetaut. Nach dem vollständigen Auftauen erfolgte unter Rühren die Zugabe von Aqua dem. ad 300,0 g. Durch diese Behandlung kommt es zum vollständigen Brechen der Dispersion, wobei sich über einem lockeren weißen Bodensatz ein klarer Überstand bildet. (Eine eventuell noch vorhandene leichte Trübe läßt sich durch Schwebestoffabtrennung mittels einer Absaugvorrichtung klären.) Der Überstand wurde dekantiert und durch die gleiche Menge Aqua dem. ersetzt und damit der Rückstand ca. 1 Minute gewaschen. Abtrennen, Ergänzen und Waschen wurde so oft (9 mal) wiederholt, bis die letzten beiden abgetrennten wässerigen Waschmedien jeweils emulgatorfrei waren. (Anstelle der Zugabe der jeweils 10-fachen Menge an Waschwasser ist es auch möglich, 15 mal mit der dreifachen Menge Wasser zu waschen. Auch dann ist das letzte Waschwasser nachweislich emulgatorfrei.)

## Patentansprüche

1. Wirkstofffreigabesystem zur kontrollierten Freigabe einer pharmazeutisch wirksamen Substanz bestehend aus wenigstens einer phamakologisch wirksamen Substanz, einem Trägermaterial und gegebenenfalls einem Hilfsstoff, dadurch gekennzeichnet, daß das Trägermaterial auf einem Emulsionspolymerisat basiert, das unter Anwendung eines Extraktionsmittels, in dem das Emulsionspolymerisat selbst nicht löslich ist, von Emulgatoren oder Tensiden und anderen in dem Extraktionsmittel lösbaren Hilfsstoffen befreit wurde.

2. Wirkstofffreigabesystem gemäß Anspruch 1, dadurch gekennzeichnet, daß das Wirkstofffreigabesystem auf emulsionspolymerisierten Estern der Acryl - und/oder Methacrylsäure basiert und das Emulsionspolymerisat im wesentlichen frei von Emulgatoren oder Tensiden ist, die mit Wasser oder einem organischen Lösungsmittel extrahierbar sind.

3. Wirkstofffreigabesystem nach Anspruch 1, dadurch gekennzeichnet, daß das als Trägermaterial dienende Emulsionspolymerisat aus der Gruppe Polylactid, Polystyrol, Polyvinylacetat, Polyvinylpyrrolidon, Polybutadien, Polyacrylnitril, der Polyvinylester, der Polyvinylether und deren Copolymere sowie deren Mischungen ausgewählt wurde.

4. Wirkstofffreigabesystem nach Anspruch 2, dadurch gekennzeichnet, daß das als Trägermaterial dienende Emulsionspolymerisat ein Polymerisat auf der Basis eines Methyl- und/oder eines Ethylesters der Acrylsäure und/oder Methacrylsäure verkörpert.

5. Wirkstofffreigabesystem nach einem der Ansprüche 1 bis 4, dadurch gekennzeichnet, daß es Wirkstoff Clonidin (2-[(2,6-Dichlorphenyl)imino]imidazolidin) enthält.

6. Wirkstofffreigabesystem nach einem der Ansprüche 1 bis 5, dadurch gekennzeichnet, daß es als Wirkstoff Clenbuterol (4-Amino-α-[(tert.-butylamino)- methyl]-3,5-dichlorbenzylalkohol) enthält.

7. Verfahren zur Herstellung eines Wirkstofffreigabesystems gemäß Anspruch 1 zur kontrollierten Freigabe von einer pharmazeutisch wirksamen Substanz, dadurch gekennzeichnet, daß man das Emulsionspolymerisat - ggf. nach dem Enffernen flüchtiger Bestandteile - mit einem Extraktionsmittel behandelt, in dem sich das Polymerisat selbst nicht bzw. nicht wesentlich löst und Emulgatoren bzw. Tenside sowie andere Hilfsstoffe extrahiert und ein bzw. mehrere derart hergestellte Emulsionspolymerisat(e) mit einem oder mehreren Wirkstoff(en) auf an sich bekannte Weise belädt.

## Claims

1. Active substance release system for the controlled release of a pharmaceutically active substance comprising at least one pharmacologically active substance, a carrier material and optionally an excipient, characterised in that the carrier material is based on an emulsion polymer which is freed from emulsifiers or surfactants and other adjuvants which are soluble in the extraction medium by means of an extraction medium in which the emulsion polymer itself is insoluble.

2. Active substance release system according to claim 1, characterised in that the active substance release system is based on emulsion-polymerised esters of acrylic and/or methacrylic acid, and that the emulsion polymer is substantially free from emulsifiers or surfactants which are extractable with water or an organic solvent.

3. Active substance release system according to claim 1, characterised in that the emulsion polymer serving as carrier material is selected from the group comprising polylactide, polystyrene, polyvinylacetate, polyvinylpyrrolidone, polybutadiene, polyacrylonitrile, the polyvinylesters, the polyvinylethers and copolymers thereof as well as the mixtures thereof.

4. Active substance release system according to claim 2, characterised in that the emulsion polymer used in the carrier material incorporates a polymer based on a methyl and/or ethyl ester of acrylic and/or methacrylic acid.

5. Active substance release system according to one of claims 1 to 4, characterised in that it contains as active substance clonidine (2-[(2,6-dichlorophenyl)-imino]imidazolidine).

6. Active substance release system according to one of claims 1 to 5, characterised in that it contains as active substance clenbuterol (4-amino-α-[(tert.-butylamino)-methyl]-3,5-dichlorobenzyl alcohol).

7. Process for preparing an active substance release system according to claim 1 for the controlled release of a pharmaceutically active substance, characterised in that the emulsion polymer - optionally after the removal of volatile components - is treated with an extraction agent in which the polymer itself is insoluble or substantially insoluble and emulsifiers or surfactants and other adjuvants are extracted and one or more emulsion polymers prepared in this way is or are charged with one or more active substances in a manner known per se.

## Revendications

1. Système de libération de principe actif pour la libération contrôlée d'une substance active du point de vue pharmaceutique, consistant en au moins une substance active du point de vue pharmacologique, un support et éventuellement une substance auxiliaire, caractérisé en ce que le support est basé sur un produit de polymérisation en émulsion qui, au moyen d'un produit d'extraction dans lequel le produit de polymérisation en émulsion lui-même n'est pas soluble, a été débarrassé des émulsifiants ou des tensioactifs et des autres substances auxiliaires solubles dans le produit d'extraction.

2. Système de libération de principe actif selon la revendication 1, caractérisé en ce que le système de libération de principe actif est basé sur des esters de l'acide acrylique et/ou méthacrylique polymérisés en émulsion et le produit de polymérisation en émulsion est sensiblement exempt d'émulsifiants ou de tensioactifs qui sont extractibles avec l'eau ou un solvant organique.

3. Système de libération de principe actif selon la revendication 1, caractérisé en ce que le produit de polymérisation en émulsion qui sert de support a été choisi dans le groupe constitué par le polylactide, le polystyrène, le poly(acétate de vinyle), la polyvinylpyrrolidone, le polybutadiène, le polyacrylonitrile, les polyvinylesters, les polyvinyléthers et leurs copolymères ainsi que leurs mélanges.

4. Système de libération de principe actif selon la revendication 2, caractérisé en ce que le produit de polymérisation en émulsion qui sert de support est un produit de polymérisation à base d'un ester méthylique et/ou d'un ester éthylique de l'acide acrylique et/ou méthacrylique.

5. Système de libération de principe actif selon l'une des revendications 1 à 4, caractérisé en ce qu'il contient le principe actif clonidine (2-[(2,6-dichlorophényl)imino]-imidazolidine).

6. Système de libération de principe actif selon l'une des revendications 1 à 5, caractérisé en ce qu'il contient comme principe actif le clenbutérol (alcool 4-amino-α-[(tert.butylamino)méthyl]-3,5-dichlorobenzylique).

7. Procédé de préparation d'un système de libération de principe actif selon la revendication 1 pour la libération contrôlée d'une substance active du point de vue pharmaceutique, caractérisé en ce que l'on traite le produit de polymérisation en émulsion, éventuellement après élimination des constituants volatils, avec un produit d'extraction dans lequel le produit de polymérisation lui-même ne se dissout pas ou ne se dissout pas sensiblement et on extrait les émulsifiants ou les tensioactifs ainsi que les autres substances auxiliaires, et on charge de manière connue en soi un ou plusieurs produits de polymérisation en émulsion ainsi préparés avec un ou plusieurs principes actifs.
